# EUROPÄISCHE PATENTSCHRIFT

(11) **EP 2 589 374 B1**
(45) Veröffentlichungstag und Bekanntmachung des Hinweises auf die Patenterteilung: **24.07.2019**
(21) Anmeldenummer: 12190020.3
(22) Anmeldetag: 25.10.2012
(51) Int. Cl.: A61K 8/97, A61Q 19/00, A61Q 19/06

(54) **Verwendung von Magnolienrindenextrakt zur Verbesserung der Hautoberflächenstruktur und zur Verminderung von Cellulite**
Use of magnolia bark extract for improved skin contouring or to counteract cellulite
Utilisation d'extrait d'écorce de magnolia pour améliorer l'aspect de la peau ou lutter contre la cellulite

(30) Priorität: 02.11.2011 DE 102011085589
(43) Veröffentlichungstag der Anmeldung: 08.05.2013
(73) Patentinhaber: Beiersdorf AG, 20253 Hamburg (DE)
(72) Erfinder: Kallmayer, Volker, 22393 Hamburg (DE); Schläger, Torsten, 22297 Hamburg (DE); Voigt, Nadine, 22111 Hamburg (DE); Holtzmann, Ursula, 22309 Hamburg (DE); Siegner, Ralf, 25421 Pinneberg (DE); Winnefeld, Marc, 22880 Wedel (DE)

(56) Entgegenhaltungen:
- DE-A1-102010 015 789
- DE-A1-102010 015 790
- US-A1- 2007 196 296
- US-A1- 2008 260 869
- Anonymous: "DaUnBee Activating Liposome Lotion(120ml) from SkinCure, Inc., Korea", , 28. September 2009 (2009-09-28), XP055098178, Gefunden im Internet: URL:http://www.ec21.com/product-details/Da UnBee-Activating-Liposome-Lotion-120ml--63 95960.html [gefunden am 2014-01-23]

## Beschreibung

Die vorliegende Erfindung betrifft die kosmetische oder dermatologische Verwendung von Magnolienrindenextrakt zur verbesserten Hautkonturierung bzw. gegen Cellulite.

Unter Kosmetik kann man alle Maßnahmen zusammenfassen, die aus ästhetischen Gründen Veränderungen an Haut und Haaren vornehmen oder zur Körperreinigung angewendet werden. Kosmetik bedeutet also, das Körperäußere zu pflegen, zu verbessern und zu verschönern, um auf sichtbare, fühlbare und riechbare Weise sowohl den Mitmenschen als auch sich selbst zu gefallen.

Ziel der Hautpflege ist es ferner, den durch tägliches Waschen verursachten Fett- und Wasserverlust der Haut auszugleichen. Dies ist gerade dann wichtig, wenn das natürliche Regenerationsvermögen nicht ausreicht. Außerdem sollen Hautpflegeprodukte vor Umwelteinflüssen, insbesondere vor Sonne und Wind, schützen und die Hautalterung verzögern.

Die chronologische Hautalterung wird z.B. durch endogene, genetisch determinierte Faktoren verursacht. In Epidermis und Dermis kommt es alterungsbedingt z.B. zu folgenden Strukturschäden und Funktionsstörungen, die auch unter den Begriff "Senile Xerosis" fallen können:
a) Trockenheit, Rauhigkeit und Ausbildung von Trockenheitsfältchen,
b) Juckreiz und
c) verminderte Rückfettung durch Talgdrüsen (z.B. nach Waschen).

Exogene Faktoren, wie UV-Licht und chemische Noxen, können kumulativ wirksam sein und z.B. die endogenen Alterungsprozesse beschleunigen bzw. sie ergänzen. In Epidermis und Dermis kommt es insbesondere durch exogene Faktoren z.B. zu folgenden Strukturschäden- und Funktionsstörungen in der Haut, die über Maß und Qualität der Schäden bei chronologischer Alterung hinausgehen:
d) Sichtbare Gefäßerweiterungen (Teleangiektasien, Cuperosis);
e) Schlaffheit und Ausbildung von Falten;
f) lokale Hyper-, Hypo- und Fehlpigmentierungen (z.B. Altersflecken) und
g) vergrößerte Anfälligkeit gegenüber mechanischem Stress (z.B. Rissigkeit).

Die vorliegende Erfindung betrifft insbesondere Produkte zur Pflege der auf natürliche Weise gealterten Haut, sowie der unter a), e) und g) aufgeführten Phänomene.

Produkte zur Pflege erschlaffter, insbesondere gealterter Haut sind an sich bekannt. Sie enthalten z.B. Retinoide (Vitamin A-Säure und/oder deren Derivate) bzw. Vitamin A und/oder dessen Derivate. Ihre Wirkung auf die Strukturschäden ist allerdings umfangsmäßig begrenzt. Daüber hinaus gibt es bei der Produktentwicklung erhebliche Schwierigkeiten, die Wirkstoffe in ausreichendem Maße gegen oxidativen Zerfall zu stabilisieren. Die Verwendung Vitamin A-Säure-haltiger Produkte bedingt darüber hinaus oft starke erythematöse Hautreizungen. Retinoide sind daher nur in geringen Konzentrationen einsetzbar.

Oftmals wird mit der erschlafften Haut auch eine Begleiterscheinung der Übergewichtigkeit und/oder der damit häufig einhergehenden sogenannten Cellullite verbunden.

Das Körperbewußtsein der Verbraucher ist in den vergangenen Jahren deutlich gestiegen. Dabei werden neben reinigenden und pflegenden Anwendungen auch zunehmend Maßnahmen ergriffen, um die Körpersilhouette zu verbessern. Die Cellulite - ein weit verbreitetes Phänomen - nimmt dabei eine zentrale Stellung ein. Das sichtbare Bild der Cellulite beruht auf einer Zunahme von Fettpolstern in der Subcutis (Unterhautfettgewebe), einer Bindegewebsschwäche sowie einer Minderung der Durchströmungsverhältnisse in den Blut- und Lymphbahnen. Die Ursache ist somit eine zum Teil anlagebedingte Schwächung des Bindegewebes mit gleichzeitigem Auftreten von vergrößerten Fettzellkammern infolge von Übergewicht, unausgewogener Ernährung, Bewegungsmangel. Die Entstehung der Cellulite kann ferner auf eine erhöhte Durchlässigkeit der Haargefäßwände zurückgeführt werden, die das Eindringen von Wasser ins Bindegewebe erlaubt. Daneben kann an den betroffenen Hautbereichen ein lokaler Mangel an Testosteron bestehen. Jedenfalls ist Cellulite eine Erscheinung die fast niemals bei Männern zu beobachten ist.

Aufgabe der vorliegenden Erfindung war es somit, Wege zu finden, die die Nachteile des Standes der Technik vermeiden. Insbesondere war es eine Aufgabe der vorliegenden Erfindung, Zubereitungen zur Verfügung zu stellen, die eine vorteilhafte Straffung erschlaffter Haut bewirken können.

Eine weitere Aufgabe der vorliegenden Erfindung war es, hautpflegende Zubereitungen und Zubereitungen zur Pflege der Haut zur Verfügung zu stellen, wobei die Zubereitungen das Körperäußere pflegen und verschönern sollten.

Es hat sich überraschenderweise herausgestellt, dass die kosmetische Verwendung von Magnolienrindenextrakt zur Verminderung von Cellulite, dadurch gekennzeichnet, daß der Magnolienrindenextrakt durch Extraktion der Rinde von Magnolia grandiflora, und/oder Magnolia officinalis gewonnen wird, den Nachteilen des Standes der Technik abhilft.

Die Verwendung erfindungsgemäßer Zubereitungen mit einem kosmetisch wirksamen Gehalt an Magnolienrindenextrakt - z. B. in Form der genannten Beispiele - führt in überraschender Weise zu einer erheblichen Verbesserung des Aussehens der Haut Es war überraschend, dass durch Anwenden erfindungsgemäßer Zubereitungen die Elastizität der Haut, der Haare und/oder der Nägel und daher Cellulite bzw. das Erscheinungsbild der sog. "Orangenhaut" verbessert wird.

Bevorzugt enthalten kosmetische oder dermatologische Zubereitungen gemäß der Erfindung 0,001 bis 30 Gew.-%, bevorzugt 0,1 bis 15 Gew.-%, besonders bevorzugt 0,5 bis 5 Gew.-% an Magnolienrindenextrakt, bezogen auf das Gesamtgewicht der Zubereitung.

Magnolien (Magnolia) sind eine Pflanzengattung in der Familie der Magnoliengewächse (Magnoliaceae). Diese enthält etwa 230 Arten, die aus Ostasien und Amerika stammen. Erfindungsgemäß kann der Magnolienrindenextrakt grundsätzlich allen Arten der Gattung Magnolia entnommen werden, wobei allerdings Magnolia grandiflora, und insbesondere Magnolia officinalis bevorzugt werden.

Magnolien-Rinde wird in der Traditionellen Chinesischen Medizin (TCM) vor allem bei "Stagnation of qi" (low energy), emotionalem Stress, Entzündungen, innerer Unruhe und Angstzuständen eingesetzt.

Die Herstellung des verwendeten Extraktes erfolgt mit Hilfe von superkritischem CO₂ oder Ethanol oder einer Ethanol / Wasser Mischung.

Magnolol ist durch folgende Struktur gekennzeichnet:

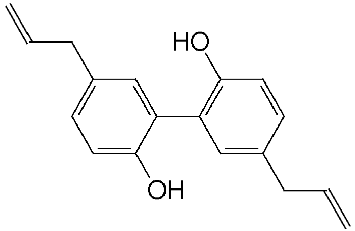

Honokiol ist durch folgende Struktur gekennzeichnet:

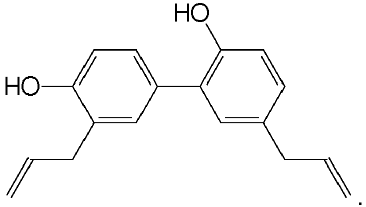

Die kosmetischen oder dermatologischen Zubereitungen gemäß der Erfindung können wie üblich zusammengesetzt sein und zur Behandlung, der Pflege und der Reinigung der Haut und/oder der Haare und als Schminkprodukt in der dekorativen Kosmetik dienen. Sie enthalten bevorzugt 0,001 Gew.-% bis 10 Gew.-%, bevorzugt 0,05 Gew.-% bis 5 Gew.-%, insbesondere 0,1 - 2,0 Gew.-%, bezogen auf das Gesamtgewicht der Zubereitungen, an Magnolienrindenextrakt.

Emulsionen sind vorteilhafte Darreichungsformen im Sinne der vorliegenden Erfindung, z.B. in Form einer Creme, einer Lotion, einer kosmetischen Milch sind vorteilhaft und enthalten z.B. Fette, Öle, Wachse und/oder andere Fettkörper, sowie Wasser und einen oder mehrere Emulgatoren, wie sie üblicherweise für einen solchen Typ der Formulierung verwendet werden.

Medizinische topische Zusammensetzungen im Sinne der vorliegenden Erfindung enthalten in der Regel ein oder mehrere Medikamente in wirksamer Konzentration. Der Einfachheit halber wird zur sauberen Unterscheidung zwischen kosmetischer und medizinischer Anwendung und entsprechenden Produkten auf die gesetzlichen Bestimmungen der Bundesrepublik Deutschland verwiesen (z.B. Kosmetikverordnung, Lebensmittel- und Arznei mittelgesetz).

Es ist dabei ebenfalls von Vorteil, den erfindungsgemäß verwendeten Wirkstoff als Zusatzstoff zu Zubereitungen zu geben, die bereits andere Wirkstoffe für andere Zwecke enthalten.

Sofern die kosmetische oder dermatologische Zubereitung im Sinne der vorliegenden Erfindung eine Lösung oder Emulsion oder Dispersion darstellt, können als Lösungsmittel verwendet werden:
- Wasser oder wäßrige Lösungen
- Öle, wie Triglyceride der Caprin- oder der Caprylsäure, vorzugsweise aber Rizinusöl;
- Fette, Wachse und andere natürliche und synthetische Fettkörper, vorzugsweise Ester von Fettsäuren mit Alkoholen niedriger C-Zahl, z.B. mit Isopropanol, Propylenglykol oder Glycerin, oder Ester von Fettalkoholen mit Alkansäuren niedriger C-Zahl oder mit Fettsäuren;
- Alkohole, Diole oder Polyole niedriger C-Zahl, sowie deren Ether, vorzugsweise Ethanol, Isopropanol, Propylenglykol, Glycerin, Ethylenglykol, Ethylenglykolmonoethyl- oder -monobutylether, Propylenglykolmonomethyl, -monoethyl- oder - monobutylether, Diethylenglykolmonomethyl- oder -monoethylether und analoge Produkte.

Insbesondere werden Gemische der vorstehend genannten Lösungsmittel verwendet. Bei alkoholischen Lösungsmitteln kann Wasser ein weiterer Bestandteil sein.

Die Ölphase der Emulsionen, Oleogele bzw. Hydrodispersionen oder Lipodispersionen im Sinne der vorliegenden Erfindung wird vorteilhaft gewählt aus der Gruppe der Ester aus gesättigten und/oder ungesättigten, verzweigten und/oder unverzweigten Alkancarbonsäuren einer Kettenlänge von 3 bis 30 C-Atomen und gesättigten und/oder ungesättigten, verzweigten und/oder unverzweigten Alkoholen einer Kettenlänge von 3 bis 30 C-Atomen, aus der Gruppe der Ester aus aromatischen Carbonsäuren und gesättigten und/oder ungesättigten, verzweigten und/oder unverzweigten Alkoholen einer Kettenlänge von 3 bis 30 C-Atomen. Solche Esteröle können dann vorteilhaft gewählt werden aus der Gruppe Isopropylmyristat, Isopropylpalmitat, Isopropylstearat, Isopropyloleat, n-Butylstearat, n-Hexyllaurat, n-Decyloleat, Isooctylstearat, Isononylstearat, Isononylisononanoat, 2-Ethylhexylpalmitat, 2-Ethylhexyllaurat, 2-Hexyldecylstearat, 2-Octyldodecylpalmitat, Oleyloleat, Oleylerucat, Erucyloleat, Erucylerucat sowie synthetische, halbsynthetische und natürliche Gemische solcher Ester, z.B. Jojobaöl.

Ferner kann die Ölphase vorteilhaft gewählt werden aus der Gruppe der verzweigten und unverzweigten Kohlenwasserstoffe und -wachse, der Silkonöle, der Dialkylether, der Gruppe der gesättigten oder ungesättigten, verzweigten oder unverzweigten Alkohole, sowie der Fettsäuretriglyceride, namentlich der Triglycerinester gesättigter und/oder ungesättigter, verzweigter und/oder unverzweigter Alkancarbonsäuren einer Kettenlänge von 8 bis 24, insbesondere 12 - 18 C-Atomen. Die Fettsäuretriglyceride können beispielsweise vorteilhaft gewählt werden aus der Gruppe der synthetischen, halbsynthetischen und natürlichen Öle, z.B. Olivenöl, Sonnenblumenöl, Sojaöl, Erdnußöl, Rapsöl, Mandelöl, Palmöl, Kokosöl, Palmkernöl und dergleichen mehr.

Auch beliebige Abmischungen solcher Öl- und Wachskomponenten sind vorteilhaft im

Die wäßrige Phase der erfindungsgemäßen Zubereitungen enthält gegebenenfalls vorteilhaft
- Alkohole, Diole oder Polyole niedriger C-Zahl, sowie deren Ether, vorzugsweise Ethanol, Isopropanol, Propylenglykol, Glycerin, Ethylenglykol, Ethylenglykolmonoethyl- oder -monobutylether, Propylenglykolmonomethyl, -monoethyl- oder -monobutylether, Diethylenglykolmonomethyl- oder -monoethylether und analoge Produkte, ferner Alkohole niedriger C-Zahl, z.B. Ethanol, Isopropanol, 1,2-Propandiol, Glycerin sowie insbesondere ein oder mehrere Verdickungsmittel, welches oder welche vorteilhaft gewählt werden können aus der Gruppe Siliciumdioxid, Aluminiumsilikate, Polysaccharide bzw. deren Derivate, z.B. Hyaluronsäure, Xanthangummi, Hydroxypropylmethylcellulose, besonders vorteilhaft aus der Gruppe der Polyacrylate, bevorzugt ein Polyacrylat aus der Gruppe der sogenannten Carbopole, beispielsweise Carbopole der Typen 980, 981, 1382, 2984, 5984, jeweils einzeln oder in Kombination.

Erfindungsgemäß verwendete Gele enthalten üblicherweise Alkohole niedriger C-Zahl, z.B. Ethanol, Isopropanol, 1,2-Propandiol, Glycerin und Wasser bzw. ein vorstehend genanntes Öl in Gegenwart eines Verdickungsmittels, das bei ölig-alkoholischen Gelen vorzugsweise Siliciumdioxid oder ein Aluminiumsilikat, bei wäßrig-alkoholischen oder alkoholischen Gelen vorzugweise ein Polyacrylat ist.

Feste Stifte enthalten z.B. natürliche oder synthetische Wachse, Fettalkohole oder Fettsäureester.

Übliche Grundstoffe, welche für die Verwendung als kosmetische Stifte im Sinne der vorliegenden Erfindung geeignet sind, sind flüssige Öle (z.B. Paraffinöle, Ricinusöl, Isopropylmyristat), halbfeste Bestandteile (z.B. Vaseline, Lanolin), feste Bestandteile (z.B. Bienenwachs, Ceresin und Mikrokristalline Wachse bzw. Ozokerit) sowie hochschmelzende Wachse (z.B. Carnaubawachs, Candelillawachs)

Als Treibmittel für aus Aerosolbehältern versprühbare kosmetische und/oder dermatologische Zubereitungen im Sinne der vorliegenden Erfindung sind die üblichen bekannten leichtflüchtigen, verflüssigten Treibmittel, beispielsweise Kohlenwasserstoffe (Propan, Butan, Isobutan) geeignet, die allein oder in Mischung miteinander eingesetzt werden können. Auch Druckluft ist vorteilhaft zu verwenden.

### Wirkungsnachweis:

### Differenzierung von Präadipozyten in Adipozyten

Subkutane, aus dem Oberschenkelbereich unterschiedlicher Spender (LOT L041806, L080608, L052206) isolierte, humane Präadipozyten wurden von Zen-Bio Inc. (Research Triangle Park, NC) erworben. Die Kultivierung der Zellen erfolgte in Basalmedium (10% fötales Kälberserum (FCS), 2 mM L-Glutamin, 100 U/mL Penicillin und 100 µg/mL Streptomycin; Cambrex, Verviers, Belgien) für 7 Tage bei 37 °C und 5% CO₂ im Zellinkubator. Das Zellkulturmedium wurde am ersten und am vierten Tag nach der Aussaat erneuert. Nachfolgend wurden die Zellen in 96 Well-Platten passagiert (1x10⁴ Zellen pro Well). 24 Stunden nach Passage wurde am folgenden Tag durch Austausch des Zellüberstands gegen Differenzierungs-Medium (Basalmedium mit 10% fötalem Kälberserum (FCS), 2 mM L-Glutamin, 100 U/mL Penicillin und 100 µg/mL Streptomycin, 10 µg/mL Insulin, 1 µM Dexamethason, 200 µM Indometacin und 500 µM Isobutylmethylxanthin; Cambrex, Verviers, Belgien) die Differenzierung der Präadipozyten zu Adipozyten induziert.

### Bestimmung der Zellvitalität

Als Maß für die Zellvitalität wurde die endogene Esterasen-Aktivität bestimmt, um eventuelle zytotoxische Effekte des Magnolienrindenextrakts zu ermitteln. Dazu wurden 2,8 µg/mL Magnolienrindenextrakt dem Differenzierungsmedium zugegeben, die Differenzierung der Zellen parallel zu einer Kontrolle ohne Magnolienrindenextrakt induziert und 7 Tage inkubiert. Anschließend wurden die Zellen 1x mit DPBS (Dulbecco's Phosphate Buffered Saline; Cambrex, Verviers, Belgien) gespült und 20 Minuten mit 100 µL/Well Fluorescein Diacetat Lösung (15 µg/mL FDA in DPBS; SIGMA, Taufkirchen, Deutschland) inkubiert. Die Fluoreszenz (ex 491 nm, em 517 nm) wurde abschließend mit einem Infinite M200 Platten-Reader (Tecan, Crailsheim, Deutschland) bestimmt.

### Triglycerid-Akkumulation während der Adipogenese (Fettzell-Differenzierung)

In 96 Well-Platten ausgesäte Preadipozyten (s.o.) wurden 7 Tage mit Differenzierungsmedium mit 2,8 µg/mL Magnolienrindenextrakt und parallel ohne Extrakt (Kontrolle) inkubiert. Die Färbung der Triglycerid-Akkumulation während der Differenzierung wurde am 7. Tag nach der Induktion mittels AdipoRed Reagenz (Cambrex, Verviers, Belgien) gemäß den Herstellerangaben durchgeführt. Die Fluoreszenz (ex 485 nm, em 572 nm) wurde abschließend mit einem Infinite M200 Platten-Reader (Tecan, Crailsheim, Deutschland) bestimmt.

### Induktion der Adipogenese (Fettzell-Differenzierung)

In 96 Well-Platten ausgesäte Preadipozyten (s.o.) wurden 7 Tage mit Differenzierungsmedium ohne Indometacin mit 2,8 µg/mL Magnolienrindenextrakt und parallel ohne Extrakt (Kontrolle) inkubiert. Die Färbung der Triglycerid-Akkumulation während der Differenzierung wurde am 7. Tag nach der Induktion mittels AdipoRed Reagenz (Cambrex, Verviers, Belgien) gemäß den Herstellerangaben durchgeführt. Die Fluoreszenz (ex 485 nm, em 572 nm) wurde abschließend mit einem Infinite M200 Platten-Reader (Tecan, Crailsheim, Deutschland) bestimmt.

Die nachfolgenden Beispiele sollen die vorliegende Erfindung verdeutlichen.

**Beispiel 1: Gel**

| **INCI-Bezeichnung** | **Gew.-%** |
|---|---|
| Ethanol, denat. | 8,00 |
| Phenoxyethanol | 0,20 |
| Methylisothiazolinon | 0,15 |
| Methylpropandiol | 2,00 |
| Glycerin | 5,00 |
| Butylenglykol | 3,00 |
| Cyclomethicon | 5,00 |
| Magnolienrindenextrakt | 1,00 |
| Carbomer | 0,20 |
| Natrium Polyacrylat | 0,50 |
| Chondrus Crispus | 0,15 |
| Acrylates/C10-30 Alkyl Acrylate Crosspolymer | 0,25 |
| Xanthan Gummi | 0,10 |
| Natriumhydroxid | q.s. |
| Parfum | q.s. |
| Wasser | Ad 100 |

**Beispiel 2: Firming-Lotion**

| **INCI-Bezeichnung** | **Gew.-%** |
|---|---|
| Paraffinum Liquidum | 10 |
| Glycerylstearat SE | 1,5 |
| Stearinsäure | 1 |
| Glycerin | 10 |
| Carbomer | 0,2 |
| Xanthan Gummi | 1 |
| Magnolienrindenextrakt | 0,25 |
| Ethanol, denat, | 3 |
| Coenzym Q 10 | 0,03 |
| Dimethicon | 2 |
| Cyclomethicon | 3 |
| Methylisothiazolinon | 0,05 |
| Natriumhydroxid | q.s. |
| Parfum | q.s. |
| Wasser | ad 100 |

**Beispiel 3: Anti-Cellulite Creme**

| **INCI-Bezeichnung** | **Gew.-%** |
|---|---|
| PEG-40 Stearat | 1,8 |
| Cetylalkohol | 3 |
| Glycerylstearat | 3 |
| Dimethicon | 2 |
| Paraffinum Liquidum | 3 |
| Glycerin | 8 |
| Carbomer | 0,2 |
| Caprylsäure-/Caprinsäuretriglyceride | 3 |
| Magnolienrindenextrakt | 0,5 |
| EDTA | 0,2 |
| Coffein | 0,5 |
| Phenoxyethanol | 0,4 |
| Methylparaben | 0,2 |
| Natriumhydroxid | q.s. |
| Parfum | q.s. |
| Wasser | ad 100 |

**Beispiel 4: Anti-Cellulite Gel**

| **INCI-Bezeichnung** | **Gew.-%** |
|---|---|
| Natrium Polyacrylat | 0,4 |
| Chondrus Chrispus | 0,1 |
| Carbomer | 0,3 |
| Cyclomethicon | 4 |
| Glycerin | 8 |
| Dimethiconol | 2 |
| Ethanol, denat. | 10 |
| Magnolienrindenextrakt | 0,35 |
| Carnitin | 0,3 |
| Natriumhydroxid | q.s. |
| Parfum | q.s. |
| Wasser | ad 100 |

**Beispiel 5: Anti-Celluliteserum**

| **INCI-Bezeichnung** | **Gew.-%** |
|---|---|
| Acrylates/C 10-30 Alkyl Acrylate Crosspolymer | 0,5 |
| Xanthangummi | 0,2 |
| Cetearylalkohol | 1 |
| Dimeticone | 1 |
| Cyclomethicone | 5 |
| Glycerin | 4 |
| Hydrogenated Polyisobuten | 2 |
| Alcohol Denat, | 10 |
| Phenoxyethanol | 0,4 |
| Methylparaben | 0,2 |
| Magnolienrindenextrakt | 0,5 |
| Wasser | ad 100 |

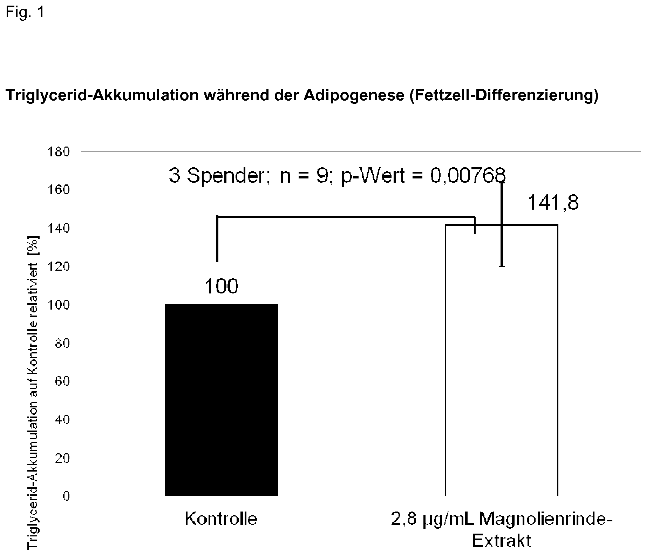

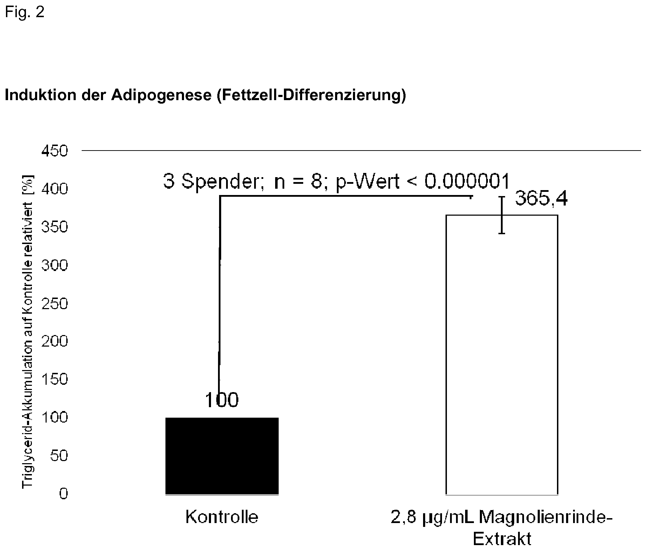

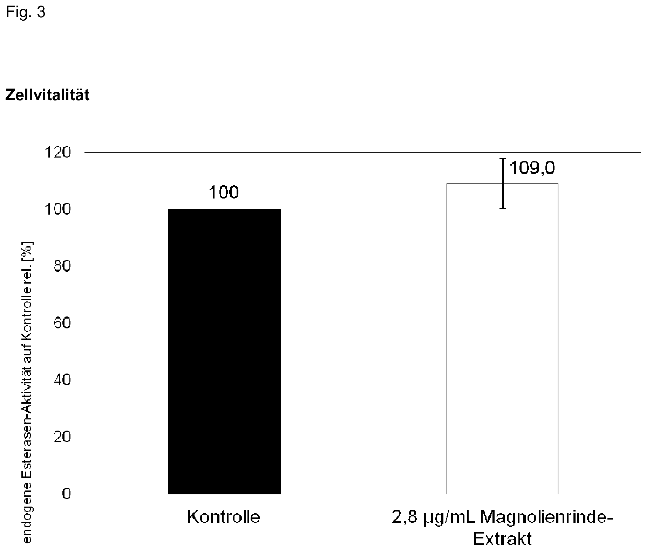

## Patentansprüche

1. Kosmetische Verwendung von Magnolienrindenextrakt zur Verminderung von Cellulite, **dadurch gekennzeichnet, daß** der Magnolienrindenextrakt durch Extraktion der Rinde von Magnolia grandiflora, und/oder Magnolia officinalis gewonnen wird.

2. Verwendung nach Anspruch 1, **dadurch gekennzeichnet, daß** der Magnolienrindenextrakt mit überkritischem CO₂ als Extraktionsmittel gewonnen wird.

3. Verwendung nach Anspruch 1, **dadurch gekennzeichnet, daß** der Magnolienrindenextrakt mit einem Wasser / Ethanol-Gemisch als Extraktionsmittel gewonnen wird.

4. Verwendung nach einem der vorstehenden Ansprüche, **dadurch gekennzeichnet, daß** der Magnolienrindenextrakt in kosmetischen oder topischen dermatologischen Zubereitungen in Konzentrationen von 0,001 - 10 Gew.-%, bevorzugt 0,05 - 5 Gew.-%, insbesondere 0,1 - 2,0 Gew.-% bezogen auf das Gesamtgewicht der Zubereitungen, vorliegt.

## Claims

1. Cosmetic use of magnolia bark extract for reducing cellulite, **characterized in that** the magnolia bark extract is obtained by extracting the bark of Magnolia grandiflora and/or Magnolia officinalis.

2. Use according to Claim 1, **characterized in that** the magnolia bark extract is obtained using supercritical CO₂ as extracting agent.

3. Use according to Claim 1, **characterized in that** the magnolia bark extract is obtained using a water/ethanol mixture as extracting agent.

4. Use according to any of the preceding claims, **characterized in that** the magnolia bark extract is present in cosmetic or topical dermatological preparations at concentrations of 0.001 - 10% by weight, preferably 0.05 - 5% by weight, especially 0.1 - 2.0% by weight, based on the total weight of the preparations.

## Revendications

1. Utilisation cosmétique d'extrait d'écorce de magnolia pour la réduction de la cellulite, **caractérisée en ce que** l'extrait d'écorce de magnolia est obtenu par l'extraction de l'écorce de Magnolia grandiflora et/ou de Magnolia officinalis.

2. Utilisation selon la revendication 1, **caractérisée en ce que** l'extrait d'écorce de magnolia est obtenu à l'aide de CO₂ supercritique en tant qu'agent d'extraction.

3. Utilisation selon la revendication 1, **caractérisée en ce que** l'extrait d'écorce de magnolia est obtenu à l'aide d'un mélange eau/éthanol en tant qu'agent d'extraction.

4. Utilisation selon l'une quelconque des revendications précédentes, **caractérisée en ce que** l'extrait d'écorce de magnolia est présent dans des préparations dermatologiques cosmétiques ou topiques en des concentrations de 0,001 -10% en poids, préférablement de 0,05-5% en poids, en particulier de 0,1-2,0% en poids, par rapport au poids total des préparations.
